Europäisches Patentamt

European Patent Office

Office européen des brevets

(11) Publication number: **0 241 119**
**A1**

## (12) EUROPEAN PATENT APPLICATION

(21) Application number: **87301693.5**

(22) Date of filing: **26.02.87**

(51) Int. Cl.4: **A61M 37/00** , A61L 15/03 , A61K 9/70

(30) Priority: **28.02.86 US 834255**

(43) Date of publication of application:
**14.10.87 Bulletin 87/42**

(84) Designated Contracting States:
**AT BE CH DE ES FR GB GR IT LI LU NL SE**

(71) Applicant: **AMERICAN HOME PRODUCTS CORPORATION**
**685, Third Avenue**
**New York, New York 10017(US)**

(72) Inventor: **Tsuk, Andrew George**
**131 Wildwood Avenue**
**Arlington Massachusetts 02174(US)**

(74) Representative: **Chettle, Adrian John et al**
**c/o John Wyeth & Brother Limited**
**Huntercombe Lane South**
**Taplow Maidenhead Berkshire, SL6 0PH(GB)**

(54) **Skin patch assembly.**

(57) The invention provides a skin patch which is storage stable but has no aluminium foil layer in the patch when applied to the skin. A skin patch assembly includes a matrix (1) incorporating a medicinal substance and secured in a sealed pouch (3, 6) formed of two sheets of aluminium foil laminate; a pull strip (4) is attached to lower sheet (3). A second pull strip (9) covers an aperture (13) in a release liner (12) secured to the upper laminate sheet (9); an adhesive film (15) covers the outer side of the aperture (13). As the pull strips (4, 9) are withdrawn the pouch (3, 6) opens to allow the matrix (1) to drop onto the adhesive film (15); the matrix (1) and film (15) constitute a skin patch for application to the skin.

FIG.1

## Skin Patch Assembly

This invention relates to adhesive patches for administration of medicinal substances through the skin.

A typical skin patch contains a medicinal substance in a sealed pouch. The pouch is opened just prior to use and the patch fixed to the skin by adhesive; the medicinal substance is gradually absorbed through the skin until the supply is exhausted. The patch is then removed for disposal and the user may substitute a fresh patch.

The skin patch assembly has several physical requirements. For example, the pouch seal must be vapour tight to protect the medication from air or moisture and to retain volatile ingredients. In the latter case the seal must also isolate the medication from the usual adhesive layer which is provided to secure the patch to the skin.

Hitherto, all commercial skin patches have used an aluminium foil film laminate because plastics films alone are not sufficiently impermeable. A typical skin patch comprises two sheets of aluminium foil film laminate heat sealed together to enclose the medication; in use one of the sheets is removed to expose the medication and adhesive, the other sheet remains as a protective layer.

Aluminium foil film laminates have several disadvantages. The aluminium foil renders the patch opaque and aesthetically unpleasant; it can cause skin irritation. The foil is conductive and may be hazardous when exposed to high electric potentials or microwave radiation.

It is an object of the present invention to provide a skin patch assembly in which the medicinal substance is sealed between sheets of aluminium foil film laminate prior to use, but which contains no aluminium foil in the patch as worn by a patient.

According to the invention there is provided a skin patch assembly comprising an aluminium foil laminate release member having a pull tab folded thereunder, an occlusive film on said member and within the perimeter thereof, a matrix on said film and incorporating a medicinal substance, an aluminium foil laminate cover releasably secured to said release member to define a sealed chamber for said film and matrix, a release sheet co-extensive with said pull tab and folded under in substantially mirror image with said release member, a release liner secured to said cover and having an aperture closed on the inner side by said release sheet, said aperture being co-extensive with said occlusive film, and a non-occlusive adhesive film secured over and around the periphery of said opening by the adhesive face thereof.

Such an assembly holds the medicinal substance in a sealed pouch of aluminium foil laminate for storage; the adhesive film which secures the patch to the skin is outside the pouch. In use the pull tab and release sheet are withdrawn; the pouch opens to allow the matrix to drop through the aperture of the release liner onto the adhesive film. The patch is then removed for application to the skin and the packaging materials disposed of. The skin patch contains no aluminium foil layer and may be coloured to be aesthetically pleasing and non-obtrusive.

Other features of the invention will be apparent from the following description of a preferred embodiment shown, by way of example only, in the accompanying drawings in which:-

Figure 1 is an exploded view of a skin patch assembly according to the invention; and

Figure 2 is a series of five views showing sequential stages in the use of the skin patch assembly of Figure 1.

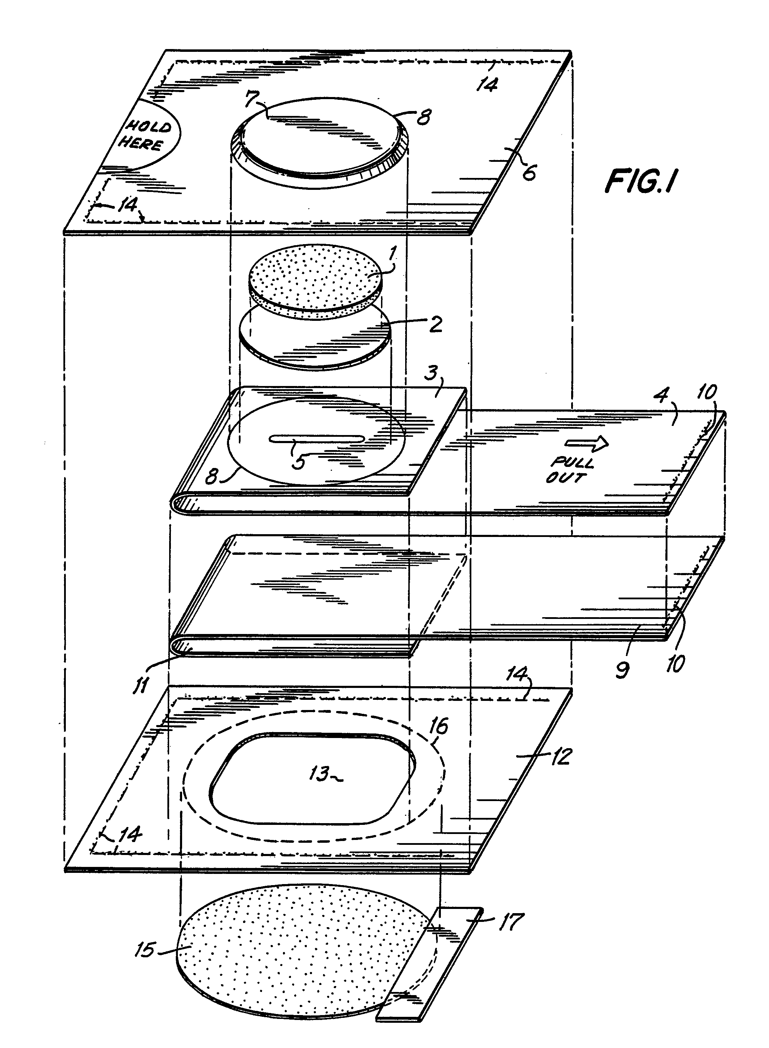

With reference to Figure 1 there is shown a matrix 1 comprising an oval piece of open cell polyurethane foam sheet, approximately 0.8mm thick, containing a liquid drug formulation. The foam holds the formulation in the manner of a sponge.

Matrix 1 is adhesively bonded to occlusive film 2, whose shape is similar to the matrix but is of slightly larger size. The occlusive film is of matte-finish polyethylene, and its role is to cover the matrix containing the liquid formulation when on the skin to prevent seepage of liquid from the treated skin, and to increase moisture retention on the treated skin.

Occlusive film 2 rests on a release member 3 which is made of a heat-sealable aluminium foil film laminate, the heat-sealable surface being the upper side of member 3 as viewed. The member 3 is folded under, as shown, to provide a pull strip 4 whose function will be explained. An optional line 5 (or string of dots) of pressure sensitive adhesive secures film 2 to member 3.

Cover 6 is also made of a heat-sealable aluminium foil film laminate, heat-sealable surface down, as shown on Figure 1. A formed cup or dome 7 in cover 6 allows room for matrix 1 and occlusive film 2. Cover 6 and release member 3 are heat-sealed together along oval line 8 to enclose the medication.

Release sheet 11 is a substantially mirror image of release member 3 and has a pull-strip 9 attached to pull-strip 4 along glue-line 10. Release sheet 11 is made of release liner material, such as light-weight paper treated on one surface to allow separation from pressure sensitive adhesives. The release surface of sheet 11 is the lower side as viewed.

Release liner 12 has the shape and size of cover 6, and can be made of heavy-weight paper, with its treated release surface down, as shown on Figure 1. Release liner 12 has an aperture 13 substantially in register with occlusive film 2 but which is larger than film 2, and is completely covered on its inner side by sheet 11. Release liner 12 is attached to cover 6 along glue-lines 14.

Non-occlusive adhesive film 15 covers the aperture 13, and outline 16 shows the edge of film 15 as it adheres to the underside of liner 12. The role of the non-occlusive adhesive film is to provide an adhesive margin around the matrix, to secure it to the skin. Porous adhesive-coated non-woven fabrics allow moisture to leave the skin from under the adhesive margin, and are preferred materials for this purpose. The adhesive side of film 15 is up, as shown on the figure.

Tab 17 helps the patient remove the patch for application, and is made of release liner material the same as liner 12, with its release surface down as shown in Figure 1.

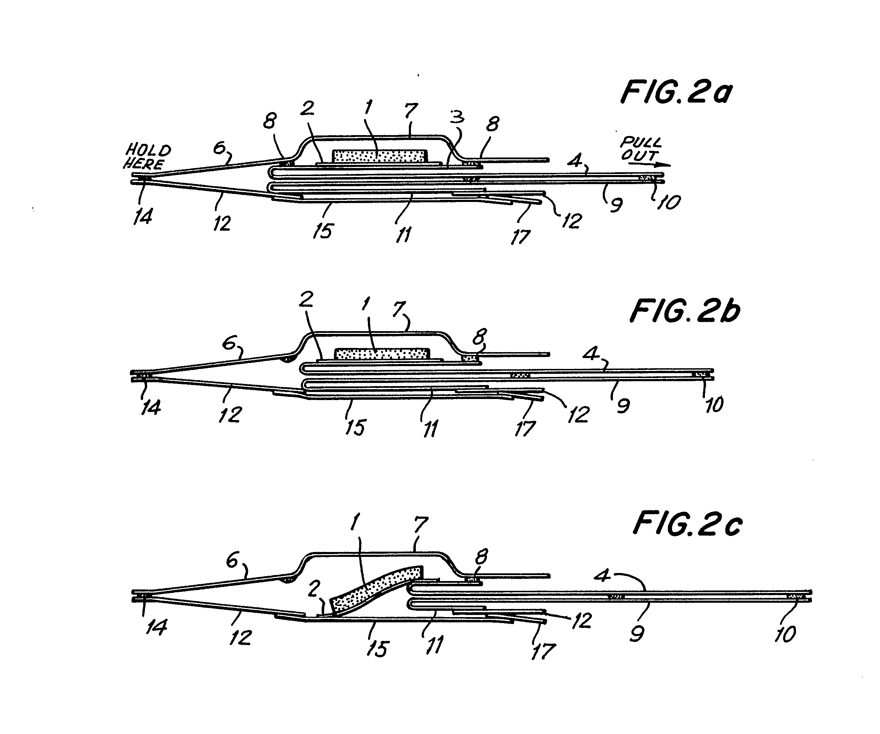

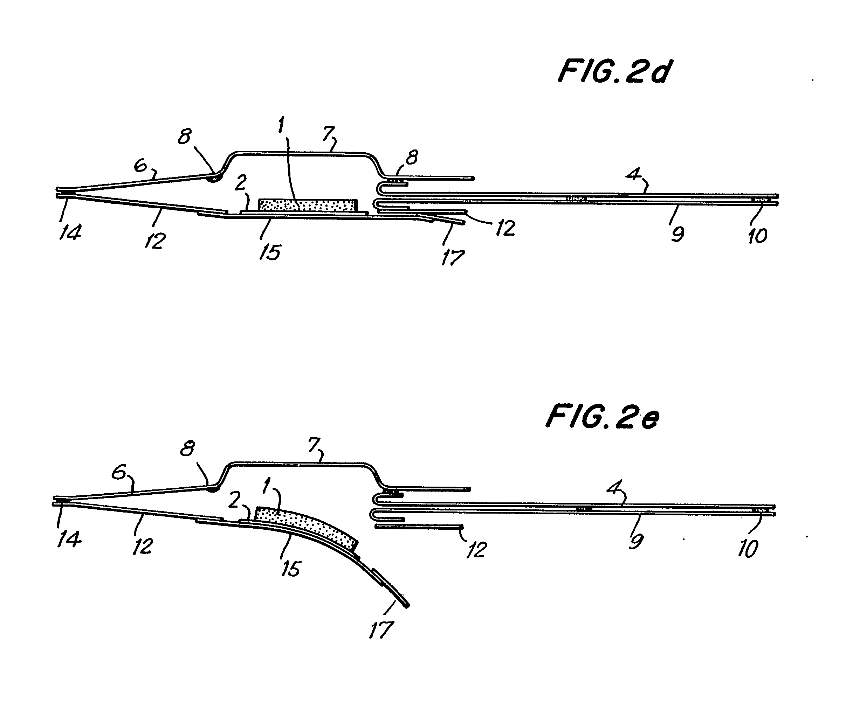

Application of the skin patch is described with reference to Figures 2a to 2e.

Figure 2a shows a transverse cross-section through the patch assembly prior to use. The skin patch is heat-sealed between cover 6 and release member 3. The pull strips 4, 9 protrude from one side of the assembly as shown.

In use, the pull strips are grasped in one hand and the assembly held in the other hand as indicated on the drawing. The strips 4, 9 are withdrawn causing the distal portions to unroll. The heat sealed line 8 is broken and the non-occlusive film 15 becomes exposed as shown in Figure 2b.

As the pull strips 4, 9 are further withdrawn, the combination of matrix 1 and occlusive film 2 drops through aperture 13 onto the adhesive surface of non-occlusive film 15 as shown in Figure 2c.

The final position of matrix 1 is shown in Figure 2d. The patient then grasps tab 17 to peel the skin patch from the packaging materials and applies the matrix 1 face down to the skin. The adhesive perimeter of film 15 secures the patch and the tab 17 is removed for disposal with the packaging materials.

The patch, as applied, contains no aluminium foil and the film 15 can be coloured to be aesthetically pleasing.

Other embodiments of the invention are possible within the scope of claims appended hereto.

## Claims

1. A skin patch assembly comprising: an aluminium foil laminate release member (3) having a pull tab (4) folded thereunder, an occlusive film (2) on said member (3) and within the perimeter thereof, a matrix (1) on said film (2) and incorporating a medicinal substance, an aluminium foil laminate cover (6) releasably secured to said release member (3) to define a sealed chamber for said film (2) and matrix (1), a release sheet (9, 11) co-extensive with said pull tab (4) and folded under in substantially mirror image with said release member (3), a release liner (12) secured to said cover (6) and having an aperture (13) closed on the inner side by said release sheet (9, 11), the aperture (13) being co-extensive with said occlusive film (2), and a non-occlusive adhesive film (15) secured over and around the periphery of said opening (13) by the adhesive face thereof.

2. A skin patch according to Claim 1, wherein said matrix (1) and occlusive film (2) are secured together by adhesive.

3. A skin patch according to Claim 1 or Claim 2, wherein said occlusive film (2) is releasably secured to said release member (3).

4. A skin patch according to Claim 3, wherein said occlusive film (2) is secured by a pressure sensitive adhesive.

5. A skin patch according to any preceding claim, wherein said pull tab (4) and release sheet (9, 11) are secured together by adhesive.

6. A skin patch according to Claim 5, wherein said pull tab (4) and release sheet (9, 11) are secured at their proximal extremity.

7. A skin patch according to any preceding claim, wherein the laminate of said release member (3) and cover (6) includes a heat sealable surface layer.

8. A skin patch according to any preceding claim, wherein said cover (6) includes a dished recess (7) to receive said matrix (1) and occlusive film (2).

9. A skin patch according to any preceding claim and further including a release tab (17) protruding from said non-occlusive adhesive film (15).

10. A skin patch according to any preceding claim, wherein said matrix (1) is of open cell polyurethane foam.

FIG.1

HOLD
HERE

7
8
14
14
6

1
2

3
4
10

8
5

PULL
OUT

11
9
10

14
16
12
13
14

15
17

FIG.2a

FIG.2b

FIG.2c

## FIG.2d

## FIG.2e

## DOCUMENTS CONSIDERED TO BE RELEVANT

EP 87301693.5

| Category | Citation of document with indication, where appropriate, of relevant passages | Relevant to claim | CLASSIFICATION OF THE APPLICATION (Int. Cl.4) |
|---|---|---|---|
| A | US - A - 4 214 592 (P.JACQUET et al.) <br> * Fig. 1,2; column 6, lines 24-59 * | 1 | A 61 M 37/00 <br> A 61 L 15/03 <br> A 61 K 9/70 |
| A | US - A - 4 470 962 (A.D.KEITH et al.) <br> * Fig. 1,2; column 7, lines 29-66 * | 1 | |
| A | DE - A1 - 3 511 963 (ALLPACK IND.) <br> * Fig.; page 13, paragraph 3 - page 15 * | 1 | |
| A | US - A - 4 450 844 (R.A.QUISNO) <br> * Totality * | 1 | |
| A | US - A - 3 972 995 (A.O.TSUK et al.) <br> * Totality * | 1 | |

| | |
|---|---|
| | TECHNICAL FIELDS SEARCHED (Int. Cl.4) |
| | A 61 B 10/00 <br> A 61 K 9/00 <br> A 61 L 15/00 <br> A 61 M 35/00 <br> A 61 M 37/00 |

The present search report has been drawn up for all claims

| Place of search | Date of completion of the search | Examiner |
|---|---|---|
| VIENNA | 24-07-1987 | LUDWIG |